(19) Europäisches Patentamt
European Patent Office
Office européen des brevets

(11) EP 3 594 198 A2

(12) # EUROPEAN PATENT APPLICATION
published in accordance with Art. 153(4) EPC

(43) Date of publication:
**15.01.2020 Bulletin 2020/03**

(21) Application number: **18764100.6**

(22) Date of filing: **09.03.2018**

(51) Int Cl.:
*C07C 69/738* (2006.01) *C07D 317/30* (2006.01)
*C07D 309/32* (2006.01) *C07C 67/327* (2006.01)
*A61K 31/222* (2006.01) *A61K 31/34* (2006.01)
*A61K 31/35* (2006.01) *A61P 9/00* (2006.01)
*A61P 17/18* (2006.01) *A61P 25/28* (2006.01)
*A61P 29/00* (2006.01) *A61P 31/00* (2006.01)
*A61P 35/00* (2006.01) *A61Q 90/00* (2009.01)
*A23L 33/105* (2016.01)

(86) International application number:
**PCT/ES2018/000016**

(87) International publication number:
**WO 2018/162769 (13.09.2018 Gazette 2018/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.03.2017 ES 201700241**

(71) Applicant: **Universidad De Sevilla**
**41013 Sevilla (ES)**

(72) Inventors:
- **FERNÁNDEZ-BOLAÑOS GUZMÁN, José María**
  **41012 Sevilla (ES)**
- **MAYA CASTILLA, Inés**
  **41012 Sevilla (ES)**
- **GONZÁLEZ BENJUMEA, Alejandro**
  **41012 Sevilla (ES)**

(74) Representative: **Pons Ariño, Angel**
**Pons Patentes y Marcas Internacional, S.L.**
**Glorieta Rubén Dario 4**
**28010 Madrid (ES)**

(54) **USE OF DMSO FOR THE SYNTHESIS OF OLEACEIN AND OLEOCANTHAL**

(57) The invention can be used to double the yield of a Krapcho reaction by means of which oleacein is obtained from oleuropein, present in olive leaves. Oleacein, a dialdehyde secoiridoid present in virgin and extra virgin olive oil, has very interesting biological properties, such as anti-inflammatory and antiasthmatic properties. For the first time, the Krapcho reaction has been extended to the conversion of monoaldehyde aglycones of oleuropein and ligstroside, present in phenolic extracts of EVOO, into the corresponding oleocanthal and oleacein dialdehyde derivatives, thereby providing a very effective method for enriching phenolic extracts with dialdehyde secoiridoids, with high added value. The invention also relates to a new method for stabilising and increasing the bioavailability of oleacein and oleocanthal using acetalation and acetylation reactions.

In terms of production, the invention falls within the scope of the agriculture, food, chemical and pharmaceutical fields.

The invention is suitable for use in the activity sector of the pharmaceutical and food industries.

EP 3 594 198 A2

## Description

### Object of the Invention

The invention can be used to double the yield of a Krapcho reaction by means of which oleacein is obtained from oleuropein, present in olive leaves. Oleacein, a dialdehyde secoiridoid present in virgin and extra virgin olive oil, has very interesting biological properties, such as anti-inflammatory and antiasthmatic properties. For the first time, the Krapcho reaction has been extended to the conversion of monoaldehyde aglycones of oleuropein and ligstroside, present in phenolic extracts of EVOO, into the corresponding oleocanthal and oleacein dialdehyde derivatives, thereby providing a very effective method for enriching phenolic extracts with dialdehyde secoiridoids, with high added value. The invention also relates to a new method for stabilising and increasing the bioavailability of oleacein and oleocanthal using acetalation and acetylation reactions.

In terms of production, the invention falls within the scope of the agriculture, food, chemical and pharmaceutical fields.

The invention is suitable for use in the activity sector of the pharmaceutical and food industries.

### State of the Art

The major components of the phenolic fraction of extra virgin olive oils (EVOO) are the dialdehydes oleacein and oleocanthal, monoaldehyde aglycones of oleuropein and ligstroside, and the corresponding dialdehyde aglycones (1).

It has been described that oleocanthal exhibits beneficial properties such as anti-inflammatory activity (2;3) and anti-tumour activity (4;5). Furthermore, oleocanthal plays a neuroprotective role in the brain (6;7), being effective in reducing β-amyloid plaques, which contributes to the prevention of the Alzheimer's disease (8).

Moreover, oleacein is a potent antioxidant and good inhibitor of the enzyme 5-lipoxygenase involved in the biosynthesis of leukotrienes, therefore oleacein can be useful in the treatment of asthma and allergic rhinitis (9). It has proven to be an effective drug against diseases related to the degradation of the atherosclerotic plaques (10), and exhibits antiproliferative properties (11).

Recently, there has been described a method for converting the oleuropein, the main phenolic compound present in olive leaves, in oleacein using the Krapcho reaction based on heating in DMSO containing NaCl and water. The published yield for this reaction is 20.5 %. It has also been described that oleacein is significantly broken down during chromatographic purification both in silica gel and in reverse phase (12).

Oleocanthal can be isolated from EVOO (13) or prepared from D-lyxose (14). Oleacein can also be synthesised from D-lyxose (14).

<u>References</u>

(1) Diamantakos P et al. Oleokoronal and oleomissional: new major phenolic ingredients of extra virgin olive oil. Olivae 2015, 122, 23

*(2)* Parkinson L & Keast R. Oleocanthal, a phenolic derived from virgin olive oil: a review of the beneficial effects on inflammatory disease. Int J Mol Sci 2014, 15, 12323

*(3) Peyrot Des Gachons C et al. Use of the irritating main oleocanthal in olive oil, as well as structurally and functionally similar compounds,* WO2006122128 A2

*(4)* Hodge AM et al. Foods, nutrients and prostate cancer. Cancer Cause Control 2004, 15, 11

*(5)* LeGendre O et al. (-)-Oleocanthal rapidly and selectively induces cancer cell death via lysosomal membrane permeabilization, Mol Cell Oncol 2015, 2, e1006077-1-8

*(6)* Heneka et al. Neuroinflammation in Alzheimer's disease, Lancet Neurol 2015, 14, 388

*(7) Theoharides TC, Anti-inflammatory compositions for treating neuroinflammation,* US2013115202 A1

*(8)* Abuznait et al. Olive oil-derived oleocanthal enhances β-amyloid clearance as a potential neuroprotective mechanism against Alzheimer's disease: In vitro and in vivo studies, ACS Chem Neurosci 2013, 4, 973

*(9)* Vougogiannopoulou et al. One-step semisynthesis of oleacein and the determination as a 5-lipoxygenase inhibitor, J Nat Prod 2014, 77, 441

*(10) Czerwinska ME et al. Oleacein for treating or preventing diseases resulting from atherosclerotic plaques,* US20160008311

*(11)* Corona et al. Inhibition of p38/CREB phosphorylation and COX-2 expression by olive oil polyphenols underlies their anti-proliferative effects. Biochem Biophys Res Commun 2007, 362, 606

*(12)* Karkoula et al. Direct measurement of oleocanthal and oleacein levels in olive oil by quantitative 1H NMR. Establishment of a new index for the characterization of extra virgin olive oils. J Agric Food Chem 2012, 60, 11696
*(13)* Fogli S et al. Cytotoxic activity of oleocanthal isolated from virgin olive oil on human melanoma cells. Nutr Cancer 2016, 68, 873
*(14)* Smith III AB et al. Syntheses of (-)-oleocanthal, a natural NSAID found in extra virgin olive oil, the (-)-deacetoxy-oleuropein aglycone, and related analogues. J Org Chem 2007, 72, 6891

**Figures**

Figure 1.- Structures of diacylated oleacein 2 and diacylated dehydrated oleuroside aglycone (DOA) 3.
Figure 2.- Krapcho demethoxycarbonylation reaction on oleuropein 1 followed by acylation, chromatographic separation and subsequent deacylation
Figure 3.- Krapcho demethoxycarbonylation reaction on monoaldehyde aglycones of the oleuropein 6 and ligstroside 7 followed by acylation.
Figure 4. - Chemoselective monoacetalation reactions on the unconjugated carbonyl of acylated oleocanthal 9 and diacylated oleacein 2.

**Description of the Invention**

The original method of converting oleuropein into oleacein has been improved using the Krapcho demethoxycarbonylation reaction in wet DMSO. With this invention, based on the absence of alkali halide, the process described above is improved by substantially reducing the heating time and increasing the yield of oleacein, more than doubling said yield. In the course of the reaction it is possible to isolate a second product, Dehydrated Oleuroside Aglycone (DOA), in respect of which there is no prior art.

The reaction mixture is acylated in situ, achieving stabilisation of both oleacein and DOA, and allowing the chromatographic purification of both. There is no prior art on diacylated oleacein; where diacetylated oleacein shall be referred to as aceolein (Figure 1, R = Me).

Using the same method, monoaldehyde aglycones of oleuropein and ligstroside are converted into oleacein and oleocanthal, respectively, by means of heating in wet DMSO. The conversion can be carried out with monoaldehyde aglycones separately, or with phenolic mixtures, such as for example mixtures of phenols isolated from olive oil. This method allows enriching the mixture of phenols with the dialdehyde secoiridoids oleacein and oleocanthal, facilitating the chromatographic isolation of both by reducing the number of components of the mixture.

The acylation of this mixture enriched with phenolic dialdehydes stabilises both compounds for the chromatographic purification step. Both diacylated oleacein **2** and oleocanthal **9** can be deacylated using lipase or a basic catalyst in alcohol (ROH)

The diacylated dialdehyde derivatives have also been stabilised by converting them into monoacetyl-monoaldehyde derivatives by means of regioselective acetalation with ethyleneglycol on the unconjugated formyl group, maintaining the acyl groups on phenolic hydroxyls. More stable and lipophilic, and therefore more readily bioavailable derivatives can thereby be obtained. Subsequent deacylation leads to the 3-ethylidene acetals of oleacein and oleocanthal.

The present invention describes the method of converting different secoiridoid compounds into oleocanthal and oleacein by means of a variant of Krapcho demethoxycarbonylation in wet DMSO at a high temperature in the absence of halide (inorganic salt). This reaction can be carried out on isolated oleuropein, on extracts rich in oleuropein, on phenolic fractions extracted from virgin and extra virgin olive oil, and on isolated monoaldehyde aglycones of oleuropein and ligstroside.

This invention allows enriching phenolic extracts from olive oil with oleacein and oleocanthal through its monoaldehyde precursors, and facilitating chromatographic separation of said dialdehyde derivatives.

*Krapcho reaction on oleuropein*

The Krapcho reaction on oleuropein **1**, or extracts rich in oleuropein, in wet DMSO or DMSO-$d_6$ at a temperature greater than 140 °C in the absence of halide (inorganic salt) leads to oleacein and dehydrated oleuroside aglycone (DOA). The chromatographic purification (silica gel, among other adsorbents) of this mixture allows isolating only DOA due to the substantial breakdown of oleacein during the process. Acylation of the mixture allows both compounds to stabilise and to furthermore be more efficiently purified by means of chromatographic separation (silica gel, among other adsorbents), which leads to the isolation the new compounds **2** and **3** (Figure 2). Deacylation of **2** carried out, for example, with lipase or base in alcohol (MeOH, among other aliphatic alcohols) leads to oleacein **4** the spectroscopic data of

which coincides with that of natural oleacein; likewise, deacylation of **3** leads to dehydrated oleuroside aglycone (DOA) **5**, not previously described. This Krapcho reaction on the ligstroside gives access to-oleocanthal **8**.

*Krapcho reaction on phenolic mixtures from virgin or extra virgin olive oil*

The Krapcho reaction with phenolic mixtures from virgin or extra virgin olive oil, containing, among other phenolic compounds, monoaldehyde aglycones of ligstroside **6** and oleuropein **7**, carried out by heating in DMSO or DMSO-$d_6$ at temperatures above 90 °C leads to the conversion of these monoaldehydes into the corresponding dialdehydes **8** (oleocanthal) and **4** (oleacein), the mixture therefore being enriched with said dialdehydes (Figure 3). The chromatographic separation of both dialdehydes allows obtaining pure oleocanthal 8 with a good yield. Acylation of the mixture of 8 and **4** followed by chromatographic separation allows obtaining pure acylated oleocanthal **9** and diacylated oleacein **2**. Acetylated oleocanthal (**9** R = Me) shall be referred to as aceocanthal and diacetylated oleacein (**2**, R = Me) shall be referred to as aceolein. There is no prior art relating to **2**, or the synthesis of **9** by acylation of oleocanthal **8**, although **9** it has been prepared through an alternative route (Smith, III et al. J Org Chem 2007, 72, 6891). Deacylation of **9** for regenerating **8** is carried out, for example, with lipase or base in alcohol (MeOH, among other aliphatic alcohols).

The Krapcho reaction on the isolated aglycone monoaldehyde of ligstroside **6**, or on the isolated aglycone monoaldehyde of oleuropein **7**, allows, after removing DMSO, directly obtaining oleocanthal **8** or oleacein **4**, respectively.

*Chemoselective derivatization on the unconjugated carbonyl of acylated oleocanthal 9 or acylated oleacein 2 using an acetalation reaction*

The treatment of acylated derivatives **2** and **9** with ethyleneglycol in the presence of a strong acid as a catalyst, for example trifluoroacetic acid, leads to **10** and **11**, respectively, acetalated in the unconjugated carbonyl (Figure 4). Deacylation of these compounds is carried out with the methods mentioned above which use, for example, lipase or base as catalyst in the presence of aliphatic alcohol, which allows obtaining monoacetalated oleacein **12** and monoacetalated oleocanthal **13**. Compounds **12** and **13** can also be obtained by acetalation of oleocanthal **8** and oleacein **4**, respectively, with ethyleneglycol and acid catalysis.

**Embodiment of the Invention**

*Preparation of aceolein (2, R = Me) and acetylated dehydrated oleuroside aglycone (3, R = Me), from oleuropein (1)*

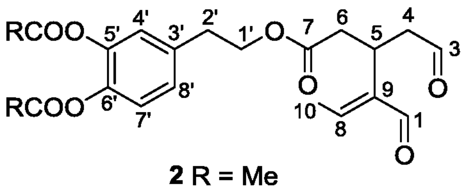

**2** R = Me

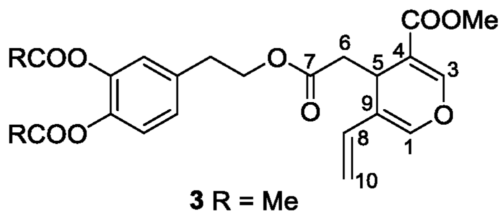

**3** R = Me

A solution of oleuropein **1** (110 mg, 0.20 mmol) in wet DMSO (3 ml) is heated at 150 °C for 5 h. The reaction mixture without removing the solvent is acetylated using $Ac_2O$ (0.4 ml) and a catalytic amount of DMAP (2 mg). Once the reaction is completed (8 h, at room temperature) the excess $Ac_2O$ is hydrolysed and concentrated to dryness at low pressure. The residue is purified by column chromatography using as eluent AcOEt-cyclohexane (1:3 $\rightarrow$ 1:1), acetylated DOA being obtained as a colourless syrup (higher $R_F$) and the aceolein (lower $R_F$) as a colourless syrup.

Data for acetylated oleacein **2** (R = Me). Yield: 38 mg, 42 % $R_F$ 0.4 (1:1 AcOEt-cyclohexane), $[\alpha]_D^{24}$ +103. $^1$H-NMR (300 MHz, $CDCl_3$): δ 9.63 (m, 1H, H-3), 9.21 (d, 1H, *J*= 2.0 Hz, H-1), 7.12 (d, 1H, *J*= 8.3 Hz, H-7'), 7.06 (dd, 1H, *J*= 8.3 Hz, *J*= 1.9 Hz, H-8'),7.02 (d, 1H, *J*= 1.9 Hz, H-4'), 6.62 (q, 1H, *J*= 7.1 Hz, H-8), 4.29□4.19 (m, 2H, H-1'), 3.63□3.55 (m, 1H, H-5), 2.97 (ddd, 1H, *J*= 18.7 Hz, J= 8.5 Hz, J= 1.2 Hz, H-4a), 2.89 (t, 2H, *J*= 6.7 Hz, H-2'), 2.74 (ddd, 1H, *J*= 18.7 Hz, *J*= 6.3 Hz, *J*= 0.9 Hz, H-4b), 2.68 (dd, 1H, *J*= 15.8 Hz, J= 8.5 Hz, H-6a), 2.60 (dd, 1H, *J*= 15.8 Hz, *J*= 6.5 Hz, H-6b), 2.28 and 2.27 (2 s, 3H each, 2 Ac), 2.04 (d, 3H, *J*= 7.1 Hz, H-10). $^{13}$C-NMR (125.7 MHz, $CDCl_3$): δ 200.6 (C-3), 195.3 (C-1), 172.0 (C-7), 168.5, 168.4 (2 OCOMe), 154.5 (C-8), 143.3 (C-9), 142.1, 140.9 (C5', C-6'), 136.8 (C-3'), 127.1 (C-8'), 124.0 (C-4'), 123.5 (C-7'), 64.5 (C-1'),46.3 (C-4), 37.0 (C-6), 34.5 (C-2'), 27.4 (C-5), 20.8 (2 OCOMe),

15.4 (C-10). HRLSI-MS: calculated for $C_{21}H_{24}NaO_8$ ([M + Na]$^+$): 427.1363, found: 427.1362.

Data for acetylated dehydrated oleuroside aglycone (DOA) 3 (R = Me). Yield: 12.5 mg, 15%, $R_F$ 0.6 (1:1 AcOEt-cyclohexane), $[\alpha]_D^{24}$ -26. $^1$H-NMR (500 MHz, $CDCl_3$): δ 7.49 (s, 1H, H-3), 7.10 (m, 2H, H-7', H-8'), 7.04 (m, 1H, H-4'), 6.53 (s, 1H, H-1), 6.14 (dd, 1H, *J*= 17.6 Hz, *J*= 11.0 Hz, H-8), 5.30 (d, 1H, *J*= 17.6 Hz, H-$10_{trans}$), 5.07 (d, 1H, *J*= 11.0 Hz, H-$10_{cys}$), 4.23 (m, 2H, *J*= 6.9 Hz, H-1'), 3.93 (t, 1H, *J*= 4.8 Hz, H-5), 3.73 (s, 3H, COOMe), 2.90 (t, 2H, *J*= 6.9 Hz, H-2'), 2.63 (dd, 1H, *J*= 14.4 Hz, J= 4.1 Hz, H-6a), 2.54 (dd, 1H, *J*= 14.4 Hz, J=5.5 Hz, H-6b), 2.29 and 2.28 (2 s, 3H each, 2 Ac). $^{13}$C-NMR (125.7 MHz, $CDCl_3$): δ 171.3 (C-7), 168.5, 168.4 (OCOMe), 166.9 (COOMe), 151.5 (C-3), 142.1 (C-5'), 141.2 (C-1), 140.8 (C-6'), 137.1 (C-3'), 127.2 (C-8), 123.9 (C-8'), 123.4 (C-4'), 123.4 (C-7'), 117.7 (C-4), 112.7 (C-10), 108.9 (C-9), 64.5 (C-1'), 51.7 (COOMe), 39.3 (C-6), 34.4 (C-2'), 27.2 (C-5), 20.8 (OCO*Me*). HRLSI-MS: calculated for $C_{23}H_{24}NaO_9$ ([M + Na]$^+$): 467.1313, found: 467.1310.

*Oleacein (4)*

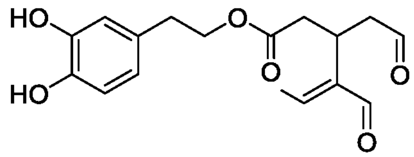

2 R = Me (12 mg, 0.038 mmol) is dissolved in MeOH (1 ml) and lipase from *Candida Antarctica* Novozyme 435 (12 mg) is added. The mixture is heated at 40 °C for 3 h. Once the reaction has ended, it is microfiltered and concentrated to dryness, the product being obtained as an orange syrup. Yield: quant. $R_F$ 0.1 (1:1 AcOEt-cyclohexane). $^1$H-NMR (300 MHz, $CDCl_3$): δ 9.64 (m, 1H, H-3), 9.22 (d, 1H, *J*= 1.9 Hz, H-1), 6.78 (d, 1H, *J*= 8.0 Hz, H-7'), 6.71 (d, 1H, *J*= 1.9 Hz, H-4'), 6.64 (q, 1H, *J*= 7.0 Hz, H-8), 6.54 (dd, 1H, *J*= 8.0 Hz, *J*= 1.9 Hz, H-8'), 4.17 (m, 2H, H-1'), 3.69 (m, 1H, H-5), 2.92 (ddd, 1H, *J*= 19.2 Hz, *J*= 8.5 Hz, *J*= 1.2 Hz, H-6a), 2.81-2.62 (m, 5H, H-4, H-2' and H-6b), 2.05 (d, 3H, *J*= 7.1 Hz, H-10).

*Dehydrated oleuroside aglycone (DOA, 5)*

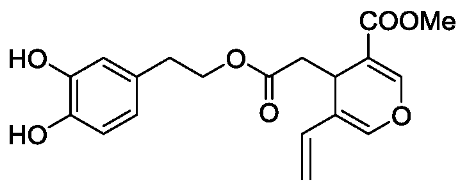

3 R = Me (32 mg, 0.072 mmol) is dissolved in MeOH (1 ml) and lipase from *Candida Antarctica* Novozyme 435 (32 mg) is added. The mixture is stirred at room temperature for 4 h. Once the reaction has ended, it is microfiltered and concentrated to dryness, the product being obtained as an orange syrup. Yield: quant. $R_F$ 0.45 (1:1 AcOEt-cyclohexane). $^1$H-NMR (300 MHz, $CDCl_3$): δ 7.52 (s, 1H, H-3), 6.78 (d, 1H, *J*= 8.0 Hz, H-7'), 6.77 (d, 1H, *J*= 2.0 Hz, H-4'), 6.61 (dd, 1H, *J*= 8.0 Hz, *J*= 2.0 Hz, H-8'), 6.57 (s, 1H, H-1) 6.15 (dd, 1H, *J*= 17.6 Hz, *J*= 11.1 Hz, H-8), 5.35 (d, 1H, *J*= 17.6 Hz, H-$10_{trans}$), 5.08 (d, 1H, *J*= 11.1 Hz, H-$10_{cys}$), 4.14 (m, 2H, H-1'), 3.95 (dd, 1H, *J*= 4.1 Hz, *J*= 5.5 Hz H-5), 3.77 (s, 3H, COO*Me*), 2.80 (t, 2H, *J*= 6.9 Hz, H-2'), 2.58 (dd, 1H, *J*= 14.4 Hz, *J*= 4.1 Hz, H-6a), 2.54 (dd, 1H, *J*= 14.4 Hz, *J*= 5.5 Hz, H-6b).

*Acetylated oleocanthal (9 R = Me)*

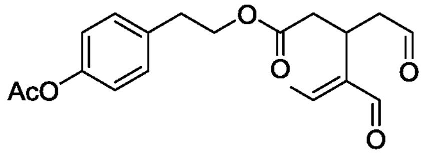

**8** (100 mg, 0.33 mmol) is dissolved in a 1:1 mixture of $Ac_2O$/Py (v/v) cooled at 0 °C. After 15 min, it is left under stirring at room temperature overnight. $Ac_2O$ is hydrolysed and concentrated to dryness at low pressure, and the residue is purified by column chromatography (AcOEt-cyclohexane 1:2), a colourless syrup being obtained. Yield: quant. $^1$H-NMR (300 MHz, $CDCl_3$): δ 9.63 (m, 1H, H-3), 9.21 (d 1H, *J*= 2.0 Hz, H-1), 7.19 (m, 2H, H-4', H-8'), 7.01 (m, 2H, H-5', H-7'), 6.61 (q, 1H, *J*= 7.1 Hz, H-8), 4.24 (m, 2H, H-1'), 3.61 (m, 1H, H-5), 2.97 (ddd, 1H, *J*= 18.3 Hz, *J*= 8.5 Hz, *J*= 1.1 Hz, H-4a), 2.89 (t, 2H, *J*= 6.9 Hz, H-2'), 2.74 (dd, 1H, *J*= 18.3 Hz, *J*= 8.5 Hz, H-4b), 2.70 (dd, 1H, *J*= 15.8 Hz, *J*= 8.4 Hz, H-6a), 2.60 (dd, 1H, *J*= 15.8 Hz, *J*= 6.6 Hz, H-6b), 2.29 (s, 3H, Ac), 2.05 (d, 3H, *J*= 7.1 Hz, H-10). $^{13}$C-NMR (75.5 MHz, $CDCl_3$): δ 200.5 (C-3), 195.3 (C-1), 172.0 (C-7), 169.7 (COOMe), 154.4 (C-8), 149.5 (C-6'), 143.4 (C-9), 135.5 (C-3'), 130.0 (C-4', C-8'), 121.8 (C-5', C-7'), 64.9 (C-1'), 46.4 (C-4), 37.0 (C-6), 34.5 (C-2'), 27.4 (C-5), 21.3 (COOMe), 15.3 (C-10). HRESI: calculated for $C_{19}H_{22}O_6Na$ ([M + Na]$^+$): 369.1309, found: 369.1309.

*5,6-Di-O-acetyloleacein 3-ethylidene acetal (11 R = Me)*

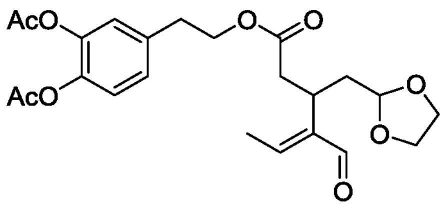

**2** R = Me (115 mg, 0.28 mmol) is dissolved in $CDCl_3$ (2 ml) and ethyleneglycol (31 μl, 0.56 mmol) and TFA (10.7 μl, 0.14 mmol) are added. The mixture is heated at 50 °C for 3 h, until the disappearance of the starting product monitored by $^1$H-NMR. Lastly, the medium is neutralised with $NaHCO_3$ and the product is purified by column chromatography (AcOEt-cyclohexane 1:2). Yield: 86 mg, 68 % $R_F$ 0.4 (1:1 AcOEt-cyclohexane). $^1$H-NMR (300 MHz, $CDCl_3$): δ 9.20 (d, 1H, *J*= 2.0 Hz, H-1), 7.09 (d, 1H, *J*= 8.2 Hz, H-7'), 7.06 (dd, 1H, *J*= 8.2 Hz, *J*= 1.9 Hz, H-8'), 6.99 (d, 1H, *J*= 1.9 Hz, H-4'), 6.56 (q, 1H, *J*= 7.1 Hz, H-8), 4.68 (dd, 1H, *J*= 6.1 Hz, *J*= 3.6 Hz, H-3), 4.19 (m, 2H, H-1'), 3.89 and 3.76 (2 m, 2 H each, $OCH_2CH_2O$), 3.28 (m, 1H, H-5), 2.85 (t, 2H, *J*= 6.8 Hz, H-2'), 2.81 (dd, 1H, *J*= 15.6 Hz, *J*= 9.6 Hz, H-6a), 2.60 (dd, 1H, *J*= 15.6 Hz, *J*= 5.5 Hz, H-6b), 2.26 and 2.25 (2 s, 3H each, 2 Ac), 2.10 (m, 1H, H-4a), 1.96 (d, 3H, *J*= 7.1 Hz, H-10), 1.83 (dt, 1H, *J*= 14.0 Hz, *J*= 5.9 Hz, H-4b). $^{13}$C-NMR (75.5 MHz, $CDCl_3$): δ 195.2 (C-1), 172.4 (C-7), 168.3, 168.2 (OCOMe), 153.0 (C-8), 144.1 (C-9), 141.9, 140.7 (C-5', C-6'), 136.8 (C-3'), 127.0 (C-8'), 123.8 (C-4'), 123.3 (C-7'), 103.2 (C-3), 64.8 and 64.7 ($OCH_2CH_2O$), 64.3 (C-1'), 37.5 (C-6), 36.0 (C-4), 34.3 (C-2'), 29.3 (C-5), 20.6 (OCOMe), 14.0 (C-10). HRLSI-MS: calculated for $C_{23}H_{28}NaO_9$ ([M + Na]$^+$): 471.1626, found: 471.1613.

*Oleacein 3-ethylidene acetal (13)*

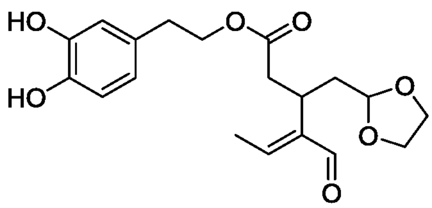

**11** (87 mg, 0.19 mmol) is dissolved in MeOH (1 ml), lipase from *Candida antarctica* Novozyme 435 (30 mg) is added and stirred at room temperature for 4 h. The mixture is microfiltered and concentrated to dryness, the product being obtained as an orange syrup. Yield: quant. $R_F$ 0.3 (1:1 AcOEt-cyclohexane)

$^1$H-NMR (300 MHz, $CDCl_3$): δ 9.21 (d, 1H, *J*= 1.9 Hz, H-1), 6.76 (d, 1H, *J*= 8.0 Hz, H-7'), 6.67 (d, 1H, *J*= 1.5

Hz, H-4'), 6.57 (q, 1H, *J*= 7.1 Hz, H-8), 6.50 (dd, 1H, *J*= 8.0 Hz, *J*= 1.5 Hz, H-8'), 4.67 (dd, 1H, *J*= 6.0 Hz, *J*= 3.6 Hz, H-3), 6.19 (bs, 2H, OH), 4.11 (m, 2H, H-1'), 3.89 and 3.75 (2 m, 2 H each, $OCH_2CH_2O$), 3.31 (m, 1H, H-5), 2.77 (dd, 1H, *J*= 15.5 Hz, *J*= 9.4 Hz, H-6a), 2.68 (t, 2H, *J*= 6.8 Hz, H-2'), 2.59 (dd, 1H, *J*= 15.5 Hz, *J*= 5.6 Hz, H-6b), 2.12 (m, 1H, H-4a), 1.97 (d, 3H, *J*= 7.1 Hz, H-10), 1.83 (dt, 1H, *J*= 14.1 Hz, *J*= 5.8 Hz, H-4b). $^{13}C$-NMR (75.5 MHz, $CDCl_3$): $\delta$ 195.7 (C-1), 172.6 (C-7), 153.8 (C-8), 144.4 (C-5'), 144.1 (C-9), 143.3 (C-6'), 129.9 (C-3'), 120.7 (C-8'), 116.4 (C-4'), 116.0 (C-7'), 103.3 (C-3), 65.3 (C-1'), 64.9, 64.8 ($OCH_2CH_2O$), 37.7 (C-6), 36.1 (C-4), 34.4 (C-2'), 29.3 (C-5), 15.1 (C-10). HRLSI-MS: calculated for $C_{19}H_{24}NaO_7$ ([M + Na]$^+$): 387.1414, found 387.1401.

*Oleocanthal 3-ethylidene acetal (12)*

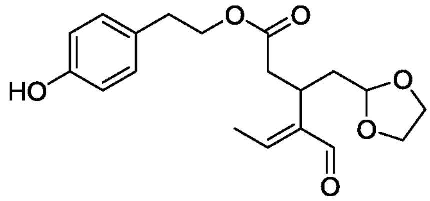

**8** (33 mg, 0.11 mmol) is dissolved in $CDCl_3$ (1 ml) and ethyleneglycol (0.22 mmol) and TFA (0.054 mmol) are added. The mixture is heated at 50 °C and monitored by $^1H$-NMR until complete conversion. The mixture is neutralised with $NaHCO_3$ and concentrated to dryness. The residue is purified by column chromatography (AcOEt-cyclohexane 1:2), a colourless syrup being obtained. Yield: 31 mg, 84 %. $^1H$-NMR (300 MHz, $CDCl_3$): $\delta$ 9.23 (d, 1H, *J*= 2.0 Hz, H-1), 7.02 (m, 2H, H-4', H-8'), 6.75 (m, 2H, H-5', H-7'), 6.57 (q, 1H, *J*= 7.1 Hz, H-8), 6.04 (bs, 1H, OH), 4.70 (dd 1H, *J*= 6.0 Hz, *J*= 3.7 Hz, H-3), 4.16 (m, 2H, H-1'), 3.86, 3.78 (2 m, 2H each, $OCH_2CH_2O$), 3.31 (m, 1H, H-5), 2.79 (t, 2H, *J*= 7.0 Hz, H-2'), 2.78 (dd, 1H, *J*= 15.7 Hz, *J*= 9.3 Hz, H-6a), 2.62 (dd, 1H, *J*= 15.7 Hz, *J*= 5.8 Hz, H-6b), 2.14 (ddd, 1H, *J*= 14.0 Hz, *J*= 9.3 Hz, *J*= 3.7 Hz, H-4a), 1.98 (d, 3H, *J*= 7.1 Hz, H-10), 1.84 (dt, 1H, *J*= 14.0 Hz, *J*= 6.0 Hz, H-4b). $^{13}C$-NMR (75.5 MHz, $CDCl_3$): $\delta$ 195.3 (C-1), 172.0 (C-7), 154.8 (C-6'), 153.5 C-8), 143.2 (C-9), 130.1 (C-4', C-8'), 129.6 (C-3'), 115.5 (C-5', C-7'), 103.4 (C-3), 65.3 (C-1'), 64.8, 64.9 ($OCH_2CH_2O$), 37.7 (C-6), 36.1 (C-4), 34.3 (C-2'), 29.3 (C-5), 15.1 (C-10). HRCI: calculated for $C_{19}H_{24}NaO_6$ ([M+Na]$^+$): 371.1465, found: 371.1456.

*4-O-Acetyloleocanthal 3-ethylidene acetal (10 R = Me)*

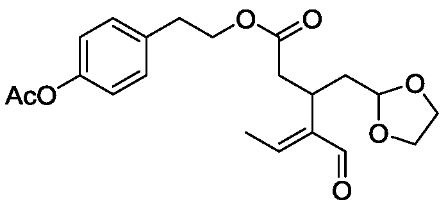

**12** (31 mg, 0.108 mmol) is dissolved in $CH_2Cl_2$ (1 ml) and $Ac_2O$ (12.2 $\mu$l, 0.16 mmol) and a catalytic amount of DMAP are added. It is stirred at room temperature overnight and hydrolysed with water. The phases are separated and the organic phase is washed with 2x5 ml of water. The organic phase is dried with $Na_2SO_4$ and concentrated to dryness. Yield: quant. $^1H$-NMR (300 MHz, $CDCl_3$): $\delta$ 9.23 (d, 1H, *J*= 2.0 Hz, H-1), 7.17 (m, 2H, H-4', H-8'), 7.00 (m, 2H, H-5', H-7'), 6.55 (q, 1H, *J*= 7.1 Hz, H-8), 4.69 (dd, 1H, *J*= 6.1 Hz, *J*= 3.6 Hz, H-3), 4.17 (m, 2H, H-1'), 3.85, 3.77 (2 m, 2H each, $OCH_2CH_2O$), 3.30 (m, 1H, H-5), 2.86 (t, 2H, *J*= 6.9 Hz, H-2'), 2.80 (dd, 1H, *J*= 15.7 Hz, *J*= 9.5 Hz, H-6a), 2.62 (dd, 1H, *J*= 15.7 Hz, *J*= 5.6 Hz, H-6b), 2.28 (s, 3H, Ac), 2.14 (ddd, 1H, *J*= 13.9 Hz, *J*= 9.2 Hz, *J*= 3.6 Hz, H-4a), 1.97 (d, 3H, *J*= 7.0 Hz, H-10), 1.84 (dt, 1H, *J*= 14.0 Hz, *J*= 5.9 Hz, H-4b). $^{13}C$-NMR (75.5 MHz, $CDCl_3$): $\delta$ 195.2 (C-1), 172.5 (C-7), 169.7 (OCOMe), 153.0 (C-8), 149.5 C-6'), 144.2 (C-9), 135.6 (C-3'), 130.0 (C-4', C-8'), 128.7 (C-5', C-7'), 103.4 (C-1), 64.9 ($OCH_2CH_2O$), 64.8 (C-1'), 64.7 ($OCH_2CH_2O$), 37.6 (C-6), 36.1 (C-4), 34.5 (C-2'), 29.4 (OCO*Me*), 21.2 (C-5), 15.0 (C-10). HRESI: calculated for $C_{23}H_{28}NaO_9$ ([M+Na]$^+$): 471.1626, found: 471.1613.

## Claims

1. Use of wet dimethylsulphoxide (DMSO) or hexadeuterated dimethylsulphoxide (DMSO-$d_6$) at temperatures above 140 °C, using conventional heating or microwaves and in the absence of inorganic salt for the conversion of oleuropein and/or ligstroside, separately or mixed with other phenolic compounds, into the oleacein and oleocanthal dialdehyde compounds, respectively.

2. Use of dimethylsulphoxide (DMSO) or hexadeuterated dimethylsulphoxide (DMSO-$d_6$) at temperatures above 90 °C, using conventional heating or microwaves, for the conversion of monoaldehyde aglycone of oleuropein and/or monoaldehyde aglycone of ligstroside, separately or mixed with other phenolic compounds, into the oleacein and oleocanthal dialdehyde compounds, respectively.

3. Use of wet dimethylsulphoxide (DMSO) or hexadeuterated dimethylsulphoxide (DMSO-$d_6$) at temperatures above 140 °C, using conventional heating or microwaves, for the conversion of oleuropein isolated (or mixed with other phenolic compounds) into dehydrated oleuroside aglycone (DOA) 5.

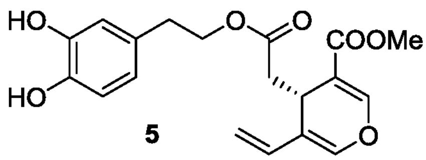

5

4. Use of dimethylsulphoxide (DMSO) or hexadeuterated dimethylsulphoxide (DMSO-$d_6$) at temperatures above 90 °C, using conventional heating or microwaves, for enriching phenolic extracts from virgin or extra virgin olive oil with oleacein and oleocanthal dialdehyde compounds.

5. Preparation of compounds of general formula **2** wherein R is selected from hydrogen (H), ($C_1$-$C_{22}$) alkyl, substituted or unsubstituted phenyl, by means of acylation of the phenolic hydroxyls of oleacein, isolated or mixed with other secoiridoids.

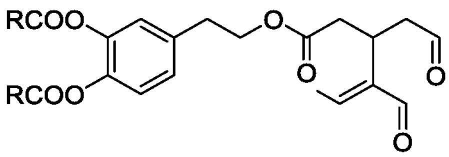

**2**

6. Preparation of compounds of general formula **9**, wherein R is selected from hydrogen (H), ($C_1$-$C_{22}$) alkyl, substituted or unsubstituted phenyl, by means of acylation of the phenolic hydroxyl of oleocanthal, isolated or mixed with other secoiridoids.

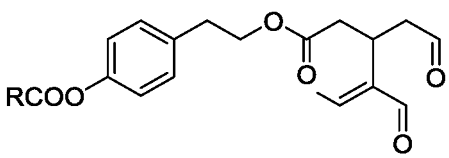

**9**

7. Preparation of compounds of general formula **3** wherein R is selected from hydrogen (H), ($C_1$-$C_{22}$) alkyl, substituted or unsubstituted phenyl, by means of acylation of the phenolic hydroxyls of dehydrated oleuroside aglycone prepared as described in claim 3.

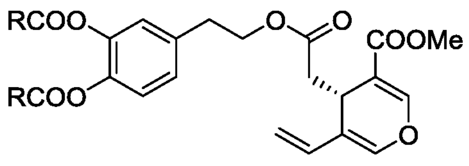

3

8. Preparation of compounds of general formula **10**, wherein R is selected from hydrogen (H), ($C_1$-$C_{20}$) alkyl, substituted or unsubstituted phenyl, by means of regioselective acetalation of acetylated oleocanthal (oleacein) with ethyleneglycol.

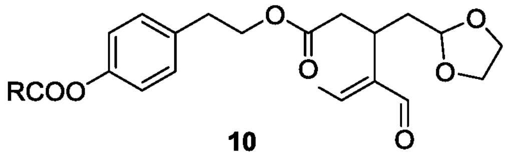

10

9. Preparation of compounds of general formula **11**, wherein R is selected from hydrogen (H), ($C_1$-$C_{22}$) alkyl, substituted or unsubstituted phenyl, by means of regioselective acetalation of acetylated oleacein (aceolein) with ethyleneglycol.

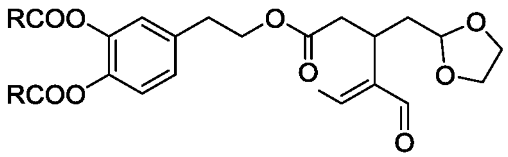

11

10. Preparation of oleocanthal 3-ethylidene acetal **12** by means of deacylation of the compound prepared according to claim 8.

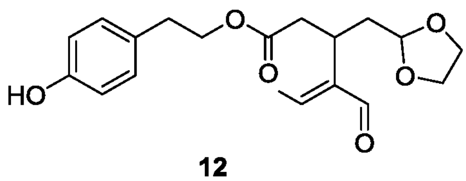

12

11. Preparation of oleacein 3-ethylidene acetal **13** by means of deacylation of the compound prepared according to claim 9.

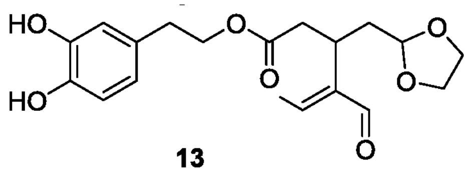

13

12. Preparation of oleocanthal 3-ethylidene acetal **12** by means of regioselective acetalation of oleocanthal with ethyleneglycol.

13. Preparation of oleocanthal 3-ethylidene acetal **13** by means of regioselective acetalation of oleacein with ethyleneglycol.

14. Use of the compounds of general formula **2**, wherein R is selected from hydrogen (H), ($C_1$-$C_{22}$) alkyl, substituted or unsubstituted phenyl prepared according to claim 5.

15. Use of the compound prepared according to claim 5, wherein R is methyl: acetylated oleacein (or aceolein).

**16.** Use of the compounds mentioned in claims 14 and 15 for the preparation of a cosmetic or pharmaceutical composition.

**17.** Use of the compounds of general formula **9**, wherein R is selected from hydrogen (H), ($C_1$-$C_{22}$) alkyl, substituted or unsubstituted phenyl, prepared according to claim 6.

**18.** Use of the compound prepared according to claim 6, wherein R is methyl: acetylated oleocanthal (or aceocanthal).

**19.** Use of the compounds mentioned in claims 17 and 18 for the preparation of a cosmetic or pharmaceutical composition.

**20.** Use of the compound of the dehydrated oleuroside aglycone (DOA), compound **5**.

**21.** Use of the compound of general formula **3**, wherein R is selected from hydrogen (H), ($C_1$-$C_{22}$) alkyl, substituted or unsubstituted phenyl.

**22.** Use of compounds **5** and **3** for the preparation of a cosmetic or pharmaceutical composition.

**23.** Use of the compounds of general formula **10**, wherein R is selected from hydrogen (H), ($C_1$-$C_{22}$) alkyl, substituted or unsubstituted phenyl.

**24.** Use of the compounds of general formula **11**, wherein R is selected from hydrogen (H), ($C_1$-$C_{22}$) alkyl, substituted or unsubstituted phenyl.

**25.** Use of compound **12** (oleocanthal 3-ethylidene acetal).

**26.** Use of compound **13** (oleacein 3-ethylidene acetal).

**27.** Use of the compounds mentioned in claims 23, 24, 25 and 26 for the preparation of a cosmetic or pharmaceutical composition.

**28.** Use of the compounds prepared according to the preceding claims for the treatment or prevention of tumour, neurodegenerative, inflammatory, cardiovascular or infectious diseases.

**29.** Use of the compounds prepared according to the preceding claims in the preparation of pharmaceutical formulations, cosmetic formulations or food compositions for the prevention of the oxidation of biomolecules, foods and cells.

**30.** Composition prepared according to any of the preceding claims, where said composition is in the form suitable for topical, oral or parental administration.

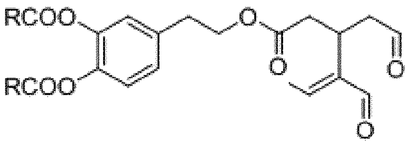

Diacylated oleacein

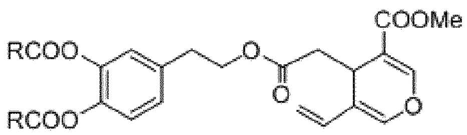

Diacylated DOA = Diacylated dehydrated oleuroside aglycone

Figure 1

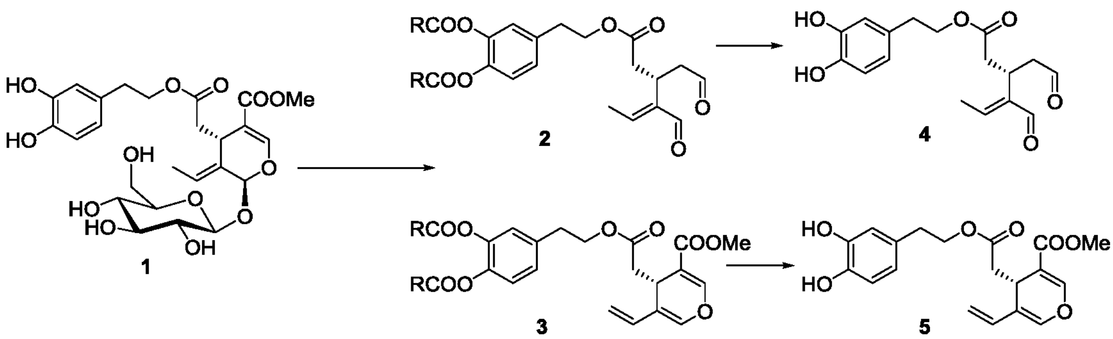

1

2

4

3

5

Figure 2

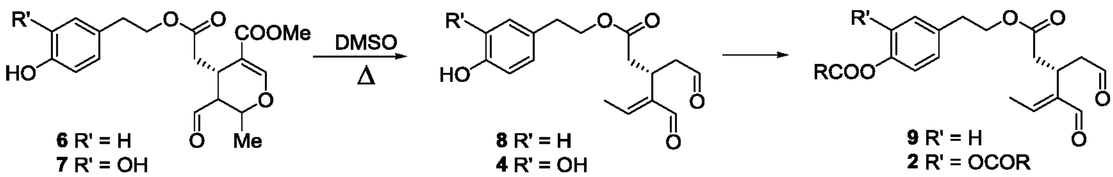


6 R' = H
7 R' = OH

8 R' = H
4 R' = OH

9 R' = H
2 R' = OCOR

Figure 3

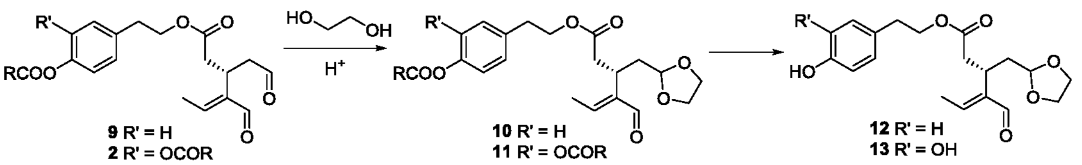


9 R' = H
2 R' = OCOR

10 R' = H
11 R' = OCOR

12 R' = H
13 R' = OH

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006122128 A2
- US 2013115202 A1
- US 20160008311 A


### Non-patent literature cited in the description


- **DIAMANTAKOS P et al.** Oleokoronal and oleomissional: new major phenolic ingredients of extra virgin olive oil. *Olivae,* 2015, vol. 122, 23
- **PARKINSON L ; KEAST R.** Oleocanthal, a phenolic derived from virgin olive oil: a review of the beneficial effects on inflammatory disease. *Int J Mol Sci,* 2014, vol. 15, 12323
- **HODGE AM et al.** Foods, nutrients and prostate cancer. *Cancer Cause Control,* 2004, vol. 15, 11
- **LEGENDRE O et al.** Oleocanthal rapidly and selectively induces cancer cell death via lysosomal membrane permeabilization. *Mol Cell Oncol,* 2015, vol. 2, e1006077-1, 8
- **HENEKA et al.** Neuroinflammation in Alzheimer's disease. *Lancet Neurol,* 2015, vol. 14, 388
- **ABUZNAIT et al.** Olive oil-derived oleocanthal enhances β-amyloid clearance as a potential neuroprotective mechanism against Alzheimer's disease: In vitro and in vivo studies. *ACS Chem Neurosci,* 2013, vol. 4, 973
- **VOUGOGIANNOPOULOU et al.** One-step semisynthesis of oleacein and the determination as a 5-lipoxygenase inhibitor. *J Nat Prod,* 2014, vol. 77, 441
- **CORONA et al.** Inhibition of p38/CREB phosphorylation and COX-2 expression by olive oil polyphenols underlies their anti-proliferative effects. *Biochem Biophys Res Commun,* 2007, vol. 362, 606
- **KARKOULA et al.** Direct measurement of oleocanthal and oleacein levels in olive oil by quantitative H NMR. Establishment of a new index for the characterization of extra virgin olive oils. *J Agric Food Chem,* 2012, vol. 60, 11696
- **FOGLI S et al.** Cytotoxic activity of oleocanthal isolated from virgin olive oil on human melanoma cells. *Nutr Cancer,* 2016, vol. 68, 873
- **SMITH III AB et al.** Syntheses of (-)-oleocanthal, a natural NSAID found in extra virgin olive oil, the (-)-deacetoxy-oleuropein aglycone, and related analogues. *J Org Chem,* 2007, vol. 72, 6891
- **SMITH, III et al.** *J Org Chem,* 2007, vol. 72, 6891